# EUROPEAN PATENT SPECIFICATION

(11) **EP 1 856 036 B1**
(45) Date of publication and mention of the grant of the patent: **14.12.2016**
(21) Application number: 05797945.2
(22) Date of filing: 29.10.2005
(51) Int. Cl.: C07C 269/04, C07C 269/00, C07C 271/44

(54) **AN EFFICIENT METHOD FOR PREPARATION OF (S)-3-[(1-DIMETHYL AMINO)ETHYL]-PHENYL-N-ETHYL-N-METHYL-CARBAMATE**
EFFIZIENTES VERFAHREN ZUR HERSTELLUNG VON (S)-3-[(1-DIMETHYLAMINO)ETHYL]-PHENYL-N-ETHYL-N-METHYLCARBAMAT
PROCEDE EFFICACE DE PREPARATION DE (S)-3-[(1-DIMETHYL AMINO)ETHYL]-PHENYL-N-ETHYL-N-METHYL-CARBAMATE

(30) Priority: 08.11.2004 IN MU12072004
(43) Date of publication of application: 21.11.2007
(73) Proprietor: Emcure Pharmaceuticals Limited, Pune 411 018 MAH (IN)
(72) Inventor: GHARPURE, Milind Moreshwar, Pune, Maharashtra 411 018 (IN); BHAWAL, Baburao Manikrao, Pune, Maharashtra 411 018 (IN); SHAH, Viral Bipinbhai, Pune, Maharashtra 411 018 (IN); ZOPE, Umesh Rewaji, Pune, Maharashtra 411 018 (IN); MEHTA, Satish Ramanlal, Pune, Maharashtra 411 018 (IN)
(74) Representative: Srinivasan, Ravi Chandran
(86) International application number: PCT/IB2005/003237
(87) International publication number: WO 2006/048720

(56) References cited:
- WO-A-2004/037771
- US-A- 4 948 807
- US-A1- 2005 096 387
- BOEZIO, ALESSANDRO A. ET AL: "Asymmetric, Catalytic Synthesis of .alpha.-Chiral Amines Using a Novel Bis(phosphine) Monoxide Chiral Ligand" JOURNAL OF THE AMERICAN CHEMICAL SOCIETY , 125(47), 14260-14261 CODEN: JACSAT; ISSN: 0002-7863, 2003, XP002367724

## Description

### FIELD OF THE INVENTION:

The present invention relates to a method for preparation of substituted phenyl carbamate and pharmaceutically acceptable salts thereof, which are of current pharmaceutical interest. The substituted phenyl carbamate and pharmaceutically acceptable salts thereof are useful to raise cholinergic activity in the central nervous system and useful in treatment of diseases such as Alzheimer's disease, Down's syndrome, Huntingdon's chorea, Friedrich's ataxia etc. 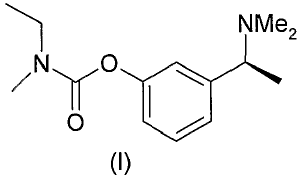 (S)-3-[(1-dimethyl amino)ethyl]- phenyl-N-ethyl-N-methyl-carbamate (I) is the active ingredient of the pharmaceutical composition referred in US 5,602,176. This compound is also used to induce selective inhibition of acetylcholinesterase activity in the brain.

### BACKGROUND OF THE INVENTION

The method for preparation of the compound (I) i.e. (S)- 3- [(1-dimethyl amino)ethyl]-phenyl-N-ethyl-N-methyl-carbamate (I) is known in literature.

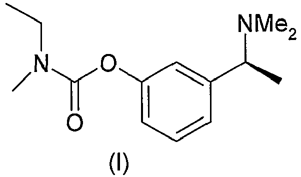

For preparation of Compound (I), as referred in WO 2004/037771, the intermediate 3-(1-dimethylaminoethyl)phenol is needed. The said intermediate is prepared by using Ti(OiPr)₄ i.e. titanium tetraisopropoxide, sodium borohydride and dimethyl amine in first step. In the second step, HBr is used. The process, due to the use of Ti(OiPr)₄, becomes expensive. Ti(OiPr)₄ is difficult to handle because it is flammable liquid and also it is irritant. Furthermore, for the work-up of the reaction, ammonia is used and moreover the titanium residue left at the end of the reaction creates effluent treatment problems and remains as environmental pollutant. Sodium borohydride is one of the expensive reducing agent used and handling of the same is hazardous and requires very skilled personnel for addition as well as work-up of the reaction. HBr causes severe bums during handling. HBr is also toxic. Also, J. Labeled Comp. and Radiophann. 1997, 39(8), 651-668 uses HBr for the same step of demethylation. Use of HBr for demethylation of methoxy function has disadvantage in the quality of final product. For industrial scale, the quantities of such reagents used are very much high and hence there is a need to avoid the use of such unsafe reagents.

EP 359647 discloses the method of preparation of morphinane analogue, wherein for demethylation of ortho-methoxy compound, sulfonic acid like methane sulfonic acid is used. However, on industrial scale such an expensive, corrosive and toxic reagent needs to be avoided.

The process of preparation as disclosed in US 4,948,807 and US 5,602,176 for compound (I) is via isocyanates or carbamoyl halides, wherein the compound (I) has the substituents on phenyl carbamate as lower alkyl.. The isocyanates, especially the one with lower alkyl group as substituents are hazardous due to their toxic properties. Use of the solvent such as dry acetonitrile or benzene are necessary for the preparation of compound (I) via isocyanates or carbamoyl halides. The industrial process using such costly, carcinogenic solvents needs to be avoided as such solvents could cause spontaneous explosions. Use of base such as sodium hydride can be avoided for the large scale reactions because it demands several safety measures, in term of storage, handling as well as processing of the reaction. Also, sodium hydride used is in the tune of 200%, which during work-up of the reaction apart from the safety hazards, causes uncontrollable exothermicity leading to the generation of various impurities.

WO 03/101917 discloses the process, wherein one of the key intermediates is N-ethyl-N-methyl-4-nitrophenyl carbamate obtained from 4-nitrophenyl chloroformate. This process does not teach the preparation of 3-(1-dimethylamino ethyl) phenol. Rather the method of preparation is by using [1-(3-methoxyphenyl) ethyl] diethylamine. The demethylation reaction is carried out by using 50% sulfuric acid and DL-methionine. The use of DL-methionine on industrial scale need to be avoided as it is skin, eye and respiratory irritant. Also, the use of DL-methionine increases the cost of production.

The reaction time is also at least 28 hrs, which increases the time to complete the whole reaction sequence till rivastigmine. Thus, obtaining 3-(1-dimethylamino ethyl) phenol is through more number of steps. Thus, there is a need to reduce the number of steps for obtaining 3-(1-dimethylamino ethyl) phenol.

Jiang et. al. (Huadong Shifan Daxue Xuebao, Ziran Kexueban (2001), (1), 61-65. / CAN 136:183572 AN 2001:429602) disclosed that the Rivastigmine can be obtained from ketone by (i) converting it into oxime (ii) reducing to amine followed by (iii) dimethylation of amine and finally (iv) converting the same into carbamate to get the racemic N-ethyl-3-[(1-dimethylamino)ethyl]-N-methyl-phenyl-carbamate (II). Thus, there are practically four functional group interconversions, only to obtain the racemic carbamate (II). The higher number of functional group interconversions lead to mainly loss of yield as well as higher cost of reagents & solvents, higher reactor occupancy, more utilities, more manpower etc. Thus, the need of efficient preparation of intermediate and in turn final rivastigmine is very essential for industrial scale.

WO 2005/061446 A1 discloses a method for preparation of Rivastigmine starting from the hydroxy phenyl ketone. The starting compound is treated with triethyl amine and ethyl methyl carbamoyl chloride to give the phenyl carbamate ketone, which when treated with dimethyl amine in the presence of sodium cyanoborohydride gives amino alkyl phenyl carbamate, which is then resolved with di-O-p-toluyl tartaric acid in aqueous methanol to give rivastigmine. This method has the drawback of employing a toxic compound like sodium cyanoborohydride, which is quite hazardous, when employed on an industrial scale. Rivastigmine thus prepared has to be crystallized several times for getting the desired purity. The repeated steps of purification reduces the overall yield considerably.

Thus, there is a need to develop the method of preparation, wherein the number of steps are reduced. Similarly, the use of Ti(OiPr)₄ is required to be avoided to make the method of preparation to be economically more viable, industrial friendly and environment friendly. Avoiding use of sodium borohydride would make the process simpler, more safe at the same time very cost efficient. Also, use of HBr need to be avoided and the method of preparation should be devoid of use of isocynate and carbamoyl chloride.

### OBJECT OF THE INVENTION

The object of the present invention is to avoid the use of Ti(OiPr)_{4,} sodium borohydride and HBr.

Another object of the invention is to provide the process, which is economical for large scale preparation.

Yet another objective is to provide the process without using isocynate and carbamoyl chloride.

Still another object of the invention is to provide rivastigmine by using carbonyl diimidazole.

### DETAILED DESCRIPTION OF THE INVENTION

A method of preparation acccording to the instant invention is according to SCHEME-1. The compound (III) is reacted with ammonia in presence of catalyst to give the product of reductive amination 3-(1-amino ethyl) phenol i.e. compound (IV). The catalyst used is Raney-Nickel. The solvent used for the reaction is alcohol such as methanol, ethanol, isopropanol, n-propanol etc. The preferable alcoholic solvent is methanol.

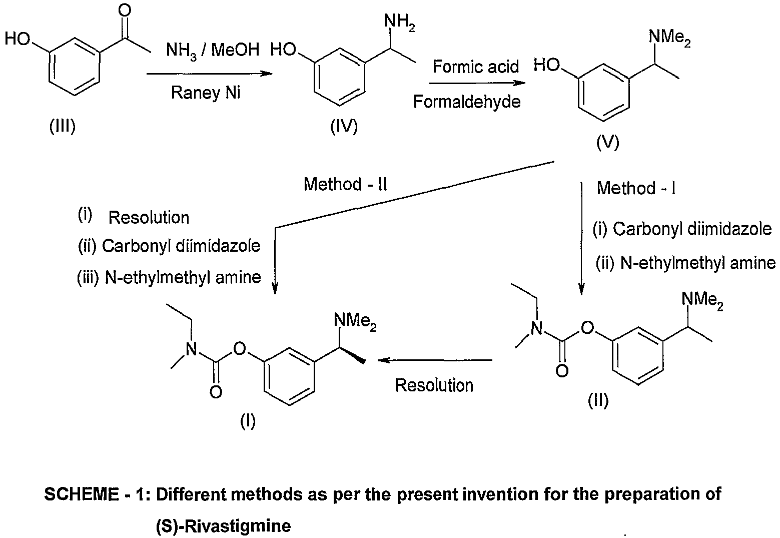

As referred in the SCHEME-1, according to the instant invention, the racemic amine (IV) i.e. 3-(1-amino ethyl) phenol is converted in to dimethylamine product (V) i.e. 3-(1-dimethylaminoethyl)phenol. Dimethylation of amine is carried out by using formic acid and formaldehyde. Thus, racemic compound (V) i.e. 3-(1-dimethylaminoethyl)phenol is obtained. Using compound (V), two methods are adopted: METHOD - 1: conversion of phenolic compound (V) into carbamate; followed by resolution of the carbamate. METHOD - 2 resolution of 3-(1-dimethylaminoethyl)phenol followed by carbamate formation.

### METHOD - 1:

### a) Preparation of 3-hydroxyl-1-phenyl ethyl amine (III).

The first step in Method-I comprises reductive amination of compound (III) with Raney nickel in the presence of ammonia.

In the present embodiment, the method for preparation of compound (IV) comprises addition of 3-hydroxy acetophenone of formula (III) to an organic solvent selected from the group comprising of hydroxylic solvents, like alcohol, water or mixture thereof. The alcohol employed is selected from the group comprising of methanol, ethanol, isopropanol, n-propanol, n-butanol etc

The preferred alcohol is methanol.

The reductive amination is carried out in alcohol either alone or in combination with water. Ammonium hydroxide followed by Raney nickel is added. The mixture is heated under pressure in an autoclave in the presence of hydrogen gas till completion of the reaction. Raney nickel was removed by filtration and the filtrate concentrated. The residue is optionally purified through the formation of their acid addition salts.

### b) Preparation of 3-(1-dimethylaminoethyl)phenol (V).

The step relates to a method for methylation of the amino group by reaction with formaldehyde in the presence of formic acid.

3-(1-dimethylaminoethyl)phenol of formula (IV) was methylated in the presence of excess formic acid and formaldehyde at reflux temperature.

The compound of formula (V) was isolated by neutralization with an aqueous solution of an inorganic base followed by extraction with an organic solvent followed by concentration of the organic layer.

### c) Preparation of 3-[1-(dimethylamino)ethyl]phenyl-N-ethyl-N-methyl carbamate (II).

This step relates to the preparation of a carbamate by reaction of ethylmethylcarbamoyl chloride with compound of formula (V) in an organic solvent and in the presence of a base.

3-[1-(dimethylamino)ethyl]phenol of formula (V) was added to an organic solvent, selected from the group comprising of aliphatic hydrocarbons, esters, chlorinated hydrocarbons, nitriles etc, preferably a nitrile like acetonitrile.

Ethylmethyl carbamoyl chloride was added to the reaction mixture.

And the reaction mixture heated till completion of reaction. The compound of formula (II) was isolated by cooling the reaction mixture to ambient temperature and extracted with an organic solvent. The organic layer was concentrated to give compound of formula (II).

The above obtained carbamate of formula (II) was resolved to get (S)-N-ethyl-3-[(1-dimethylamino)ethyl]-N-methyl-phenyl-carbamate (I).

### METHOD - 2:

3-(1-Dimethylamino ethyl) phenol is resolved using various resolving agents such as selected from the group comprising of optically active tartaric acid, di-O-tolyl tartaric acid, dibenzyl tartaric acid, mandelic acid, camphor sulfonic acid etc In the further reaction, the optically pure compound (V-A) is used for preparation of the desired carbamate i.e. (S)-N-ethyl-3-[(1-dimethylamino)ethyl]-N-methyl-phenylcarbamate (I).

The carbamate formation is carried out by various methods, wherein the agent to insert the carbonyl moiety between amine and phenolic component are different; such as carbonyldiimidazole, triphosgene, methyl carbonate etc. The carbonyl insertion reagent can be first reacted with phenolic OH group followed by reaction with amine component. Optionally, the carbonyl insertion reagent is first reacted with amine and followed by reaction with phenolic OH group.

Alternatively, 4-nitrophenyl chloroformate is reacted with 3-(1-dimethylamino ethyl) phenol and followed by reaction of the said product with N-ethylmethylamine.

Among the carbonyl inserting agents it was carbonyldiimidazole, which was found to be the reagent of choice, because reaction was faster and isolation of the product of formula (II) was easier.

Method-II is similar to Method-I, till the preparation of compound (V).

### i) Resolution of 3-(1-dimethylaminoethyl)phenol of formula (V) to give (S)- 3-(1-dimethylaminoethyl)phenol of formula (V-a).

The first step in Method-II relates to resolution of compound (V) by utilizing camphor sulphonic acid to give 3-(1-dimethylaminoethyl)phenol of formula (V) to give (S)- 3-(1-dimethylaminoethyl)phenol of formula (V-a).

### ii) Preparation of Rivastigmine.

### a) Preparation of Rivastigmine by utilizing carbonyl diimidazole and ethyl methyl amine.

The preparation of Rivastigmine comprises reaction of (S)-3-{1-(dimethylaminoethyl)phenol or its racemic mixture with ethyl methyl amine in the presence of carbonyl diimidazole (CDI). This reaction involves preparation of the carbamate derivative of compound (V-a), i.e. rivastigmine of formula (I) by insertion of the carbonyl group between the phenolic oxygen and the nitrogen atom of ethyl methyl amine.

It is pertinent to mention that carbonyl diimidazole has been employed for the first time for the preparation of rivastigmine, avoiding hazardous reagents like ethyl methyl carbamoyl chloride, isocyanates, phosgene etc.

The reaction comprises addition of (S)-3-{1-(dimethylaminoethyl)phenol (V-a) or its racemate of formula (V) to an organic solvent.

The organic solvent is selected from the group comprising of aliphatic hydrocarbons, esters, chlorinated solvents etc.

The preferred solvent is a chlorinated solvent.

The chlorinated solvent is selected from the group comprising of dichloromethane, ethylene dichloride, and chloroform.

The preferred chlorinated solvent is dichloromethane.

The amount of the chlorinated solvent employed is between 2 volumes and 20 volumes per gram of compound (V-a) or (V).

The preferred volume of the solvent employed is between 4 volumes and 10 volumes per gram of compound (V-a) or (V).

Carbonyl diimidazole is added to the reaction mixture.

The amount of carbonyl diimidazole added is between 1.5 mole and 3.0 moles per mole of compound (V-a) or (V).

The preferred amount of carbonyl diimidazole added is between 1.75 mole and 2.0 moles per mole of compound (V-a) or (V).

The reaction mixture is refluxed for duration between 10 and 15 hours and the reaction monitored by HPLC.

The reaction mixture is cooled between 0°C and 10°C, and ethyl methyl amine added. The amount of ethyl methyl amine added is between 1.0 moles and 3.0 moles per mole of compound (V-a) or (V).

The preferred amount of ethylmethyl amine added is between 1.75 moles and 2.25 moles per mole of compound (V-a) or (V).

The reaction mixture is stirred at ambient temperature for 4-6 hours.

After completion of the reaction, the mixture is quenched with water. The organic layer is separated and washed with a dilute aqueous solution of an inorganic base.

The inorganic base is selected from sodium carbonate, potassium carbonate, sodium hydroxide, potassium hydroxide etc.

The preferred inorganic base is sodium hydroxide.

The organic layer is washed with a dilute solution of a mineral acid like hydrochloric acid, sulphuric acid etc.

The preferred mineral acid is hydrochloric acid.

The organic layer is then treated with dilute solution of ammonium hydroxide and extracted with an organic solvent.

The organic solvent is selected from the group comprising of aliphatic hydrocarbons, chlorinated hydrocarbons, esters etc.

The preferred organic solvent is an aliphatic hydrocarbon selected from the group comprising of hexane, cyclohexane, heptane etc.

The organic layer after extraction is concentrated to give compound of formula (I) having an optical purity of 99% or compound of formula (II), i.e..racemic rivastigmine, which is then resolved by standard methods to give Rivastigmine of formula (I).

Thus by avoiding resolution of racemic rivastigmine of formula (II) in the last step and by employing a very much less hazardous, safe, readily available regent like carbonyl diimidazole, and ethylmethyl amine the process becomes cost-effective, environmental friendly and more suitable for industrial purpose.

### Preparation of Rivastigmine from (S)-3-{1-(dimethylaminoethyl)phenol (V-a) by employing ethylmethyl carbamoyl chloride.

The method comprises reaction of (S)-3-{1-(dimethylaminoethyl)phenol (V-a) with ethylmethyl carbamoyl chloride in the presence of a mild inorganic base or organic base.

It is pertinent to mention that prior art methods, which disclose the above reaction, disclose utilization of a strong base like sodium hydride, which is hazardous on industrial scale. Further, it is also important to note that when the desired isomer of the intermediate (S)-3-{1-(dimethylaminoethyl)phenol (V-a) is employed for preparing Rivastigmine, only half the amount of ethylmethyl carbamoyl chloride is required, thereby making the process cost-effective for industrial scale.

3-[1-(Dimethylamino)ethyl]phenol of formula (V) was added to an organic solvent, selected from the group comprising of aliphatic hydrocarbons, esters, chlorinated hydrocarbons, nitriles etc.

The preferred solvent is a nitrile.

The nitriles are selected from the group comprising of acetonitrile, propionitrile, butyronitrile etc.

The preferred solvent is acetonitrile.

The volume of acetonitrile employed is between 5 and 20 times volume of acetonitrile per gram of compound (V-a).

The preferred volume of acetonitrile is between 10 and 15 times volume of acetonitrile per gram of compound (V-a).

A base selected from the group comprising of an inorganic base or an organic base is added to the mixture.

The preferred base is an inorganic base.

The inorganic base is selected from the group comprising of a carbonate, bicarbonate, alkoxide of a alkali metal or the carbonate, bicarbonate of an alkaline earth metal.

The preferred inorganic base is the carbonate of an alkali metal.

The inorganic base is selected from the group comprising of lithium carbonate, sodium carbonate or potassium carbonate.

The preferred inorganic base is potassium carbonate.

The amount of potassium carbonate employed is between 1 .0 moles and 4.0 moles per mole of compound of formula (V-a).

The preferred amount of potassium carbonate is between 2.75 moles and 3.25 moles per mole of compound of formula (V-a).

Ethylmethyl carbamoyl chloride was added to the reaction mixture.

The amount of ethyl methyl carabamoyl chloride added is between 1.0 and 1.5 mole per mole of compound of formula (V-a).

The reaction was carried out at a temperature between 60°C and 90°C. The preferred temperature was 70°C-80°C.

After completion of the reaction, the mixture was cooled to ambient temperature and concentrated. The residue was diluted with water and extracted with an organic solvent to give the product of formula (I).

Thus the advantage lies in utilizing only half the amount of ethyl methyl carbamoyl chloride, thereby making the process cost-effective and suitable for industrial scale. The process of present invention is described herein below with reference to examples, which are illustrative only and should not be construed to limit the scope of the present invention in any manner.

### EXAMPLES

### Preparation of 3-hydroxy1-1-phenyl ethylamine (IV)

Reductive amination was carried on 3-hydroxy acetophenone (25gm) with ammonical methanol (250ml) in the presence of Raney nickel (5 gm) at 80°C and 10kg /cm2 of hydrogen pressure in an autoclave. After 12-14 hours the product was isolated by removing Raney nickel and concentrating the filtrate. The product was further purified by making its hydrochloride salt, extracting with ethyl acetate (100 ml) and neutralizing with caustic solution till pH 11-12 was attained which gave a yield of 70%.

Yield: 21.25 gms.

% Yield: 85%.

### HPLC Purity: 98-99%.

### Preparation of 3-(1-dimethylaminoethyl) phenol (V)

N-Alkylation was carried on 3-hydroxy-1-phenylethylamine (10gm) with 2 eq. of formaldehyde and 4 eq. of formic acid. The reaction was carried at 90°C for 10-12 hours. The reaction mixture was neutralized with caustic solution and extracted with ethyl acetate (3 X 50 l). Further concentration gave the desired product with 70% yield. Yield: 80-90 gms

%Yield: 70%

HPLC Purity: 97%.

### Preparation of 3-[1-(dimethylamino) ethyl] phenyl-N-ethyl-N-methylcarbamate (II)

Condensation reaction of 3-(1-dimethylaminoethyl) phenol (10 gm) and ethylmethylcarbamoyl chloride (10gm) was carried in the presence of potassium carbonate (25 gm) and acetonitrile (150 ml) at 70-80° C. After completion of reaction, the reaction mixture was quenched in water (250 ml) at 30-35° C and extracted in ethyl acetate (100ml). Evaporation of ethyl acetate gave the desired compound in 70% yield. Yield: 80-85 gms.

%Yield: 66.0%

HPLC Purity: 99%.

### Preparation of (-)-S-3-[1-(dimethylamino) ethyl] phenyl-N-ethyl-N-methylcarbamate (I)

The resolution of racemic compound (10gm) with (+)-O, O'-ditoluyltartaric acid (16gm) in methanol (20ml) and water (10ml) gave 6 gm of desired isomer under cold condition. The pure isomer was obtained after crystallization (thrice) in mixture of water and methanol (1:2). The tartrate salt (6 gm) was treated with caustic solution, extracted with dichloromethane and concentrated in vacuum to yield 2 gm of pale yellow oil.

Yield: 20-25gms.

%Yield: 20%(w/w).

HPLC Purity: 99%.

### Preparation of (S)-3-(1-dimethylaminoethyl) phenol (V-a).

3-(1-Dimethylaminoethyl) phenol (25g, 0.15mole) was added to Ethyl acetate (125ml) followed by D-(+)-10-camphor sulphonic acid (35 g, 0.15 mole). The reaction mass was heated to reflux temperature. Methanol (17ml) was added. The reaction mass was refluxed for 30 minutes and filtered at 10°C to give compound of formula (V-a). Optionally, the salt was further recrystallized from a mixture of ethyl acetate and methanol.

Yield: 6.25-7.5 gms

%Yield: 25-30%

HPLC Purity: 98%

### Preparation of Rivastigmine from (S)-3-(1-dimethylaminoethyl) phenol (V-a) by utilizing ethylmethyl carbamoyl chloride.

(S)-3-(1-dimethylaminoethyl) phenol (V-a; 25gm; 0.15mole) was reacted with Ethyl methyl carbamoyl chloride (20gm, 0.165 mole) in presence of anhydrous.potassium carbonate (31.5 gm, 0.228 moles) and acetonitrile (250ml) and heated. After completion of reaction the reaction mixture was filtered and the filtrate concentrated to give product. The product was optionally purified by acid base treatment.

Yield: 27 gm

Yield: 74%

Purity: 99% (by HPLC)

### Preparation of 3-[1-(dimethylamino) ethyl] phenyl-N-ethyl-N-methylcarbamate via CDI and ethylmethyl amine

3-(1-dimethylaminoethyl) phenol (10 gm, 0.06 moles) was added to dichloromethane (50ml) followed by addition of carbonyl diimidazole (20g, 0.123mole). The reaction mass was refluxed for 12 hours and the reaction mass was then cooled to 5-10°C. Ethylmethyl amine (7.08gms; 2.0 moles) was added to the mixture and stirred for 4-6 hours till completion of reaction. Water (100 ml) was added to quench the reaction. The dichloromethane layer was separated and treated with 10% NaOH solution (50ml) at 10-15°C. The organic layer was then treated with dilute hydrochloric acid, neutralized with ammonia solution and extracted with Hexane. The hexane layer was concentrated to give compound (I).

### Preparation of Rivastigmine hydrogen tartrate.

(-)-S-3-[1-(dimethylamino)ethyl]phenyl-N-ethyl-N-methylcarbamate (I) (100gms;1.0 mole) and L-tartaric acid (60gms; 1.0mole) were added to acetone (1000ml) and the mixture heated at 60°C for 1.0 hour to get a clear mixture, which was then cooled for complete precipitation of the tartrate salt of compound (I). The tartrate salt of compound was filtered and dried.

Yield: 135 gms.

%Yield: 85%.

HPLC Purity: 99%.

The advantage of the instant process is:
1. The method utilizes a readily available, industrially safe raw material like N,N-carbonyl diimidazole for obtaining the carabamoyl function. It has to be noted that N,N-carbonyl diimidazole has not been used before for preparing rivastigmine.
2. The reagent used for preparation of carbamate such as N-ethyl-N-methyl carbamoyl chloride are highly expensive. The reagents such as N,N-carbonyl diimidazole used for preparation of carbamate will be used only in half the quantities, especially because the undesired isomer (being absent) does not take the part in the reaction. Thus, the method of preparation is industrially cost effective.

## Claims

1. A process for the preparation of rivastigmine of formula (I) 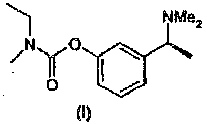 comprising
a) reacting 3-(1-dimethylaminoethyl) phenol of formula (V) with carbonyl diimidazole 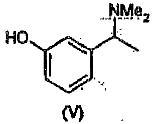
b) performing a step of reacting a product of step-a with ethylmethyl amine in an organic solvent to obtain compound (II); 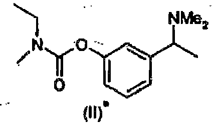
c) optionally resolving the compound (II) to yield compound (I).

2. The process as claimed in claim 1, wherein 3-(1-dimethylaminoethyl) phenol is the optically active (S)-3-(1-dimethylaminoethyl) phenol.

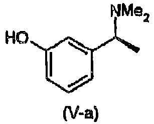

3. The process as claimed in claim 1 and 2, wherein rivastigmine of formula (I) is obtained by reacting (S)-3-(1-dimethylaminoethyl) phenol of formula V(a) with carbonyl imidazole and ethyl methyl amine in an organic solvent.

4. A process according to claims 1 and 3, wherein the amount of carbonyl diimidazole employed is between 1.5 moles and 3.0 moles per mole of compound of formula (V) or formula (Va).

5. A process according to claim 4, wherein the amount of carbonyl diimidazole employed is preferably between 1.75 moles and 2.25 moles per mole of compound of formula (V) or compound of formula (V-a).

6. A process according to claims 1 and 3, wherein the amount of ethylmethyl amine employed is between 1.0 mole and 3.0 moles per mole of compound of formula (V) or compound of formula (V-a).

7. A process according to claim 6, wherein the amount of ethylmethyl amine employed is preferably between 1.75 moles and 2.25 moles per mole of compound of formula (V) or compound of formula (V-a).

8. A process according to claim 1b and claim 3, wherein the solvent employed is selected from the group comprising of aliphatic hydrocarbon, esters, and chlorinated solvents.

9. A process according to claim 8, wherein the preferred solvent is a chlorinated hydrocarbon.

10. A process according to claim 9, wherein the chlorinated solvent is selected from the group comprising of dichloromethane, chloroform; and ethylene dichloride.

11. A process according to claim 9, wherein the chlorinated hydrocarbon is preferably dichloromethane.

12. A process according to claim 1 or claim 3, wherein when the resolving step is not required, the compound of formula (I) is isolated from the reaction mixture, by quenching the mixture with water, followed by extraction with an organic solvent and concentration of the organic layer

13. A process according to claim 1, wherein for step c), the compound of formula (I) is isolated from the reaction mixture, by quenching the mixture with water, followed by concentration of the organic layer to give compound of formula (II), which is then resolved by employing an optically active acid like D-camphor sulphonic acid.

14. A process for preparation of rivastigmine of formula (I) comprising reaction of (S)-3-(1-dimethylaminoethyl) phenol of formula (V-a) with an ethylmethyl carbamoyl chloride in an inert organic solvent at a elevated temperature and in the presence of a base selected from the group comprising of carbonates, bicarbonate, alkoxides of alkali or alkaline earth metals and isolating compound of formula (I).

15. A process according to claim 14, wherein the organic solvent is selected from the group comprising of aliphatic hydrocarbons, esters, chlorinated hydrocarbons and nitriles.

16. A process according to claim 15, wherein the preferred organic solvent is a nitrile.

17. A process according to claim 16, wherein the nitrile is selected from the group comprising of acetonitrile, propionitrile and butyronitrile.

18. A process according to claim 17, wherein the preferred nitrile is acetonitrile.

19. A process according to claim 18, wherein the volume of acetonitrile employed is between 10 and 15 volumes per gram of compound (V-a).

20. A process according to claim 14, wherein the preferred base is the carbonate of an alkali metal.

21. A process according to claim 20, wherein the base is selected from the group comprising of lithium carbonate, sodium carbonate and potassium carbonate.

22. A process according to claim 21, wherein the preferred base is potassium carbonate.

23. A process according to claim 22, wherein the amount of potassium carbonate utilized is between 1.0 and 4.0 moles per mole of compound (V-a).

24. A process according to claim 14, wherein the amount of ethylmethyl carbamoyl chloride employed for the reaction is between 1.0 and 1.5 moles per mole of compound (V-a).

25. A process according to claim 14, wherein the compound of formula (I) is isolated from the reaction mixture, by quenching the mixture with water, followed by extraction with an organic solvent and concentration of the organic layer.

## Patentansprüche

1. Prozess zur Herstellung von Rivastigmin der Formel (I) 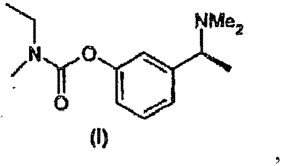 umfassend
a) Verreagieren von 3-(1-Dimethylaminoethyl)phenol der Formel (V) mit Carbonyldiimidazol 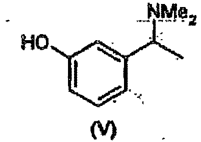
b) Ausführen eines Schritts des Verreagierens eines Produkts aus Schritt a) mit Ethylmethylamin in einem organischen Lösungsmittel, um Verbindung (II) zu gewinnen; 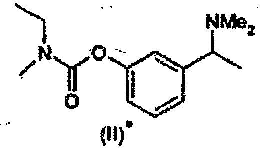
c) optional Lösen der Verbindung (II), um Verbindung (I) zu erhalten.

2. Prozess gemäß Anspruch 1, wobei 3-(1-Dimethylaminoethyl)phenol das optisch aktive (S)-3-(1-Dimethylaminoethyl)phenol ist.

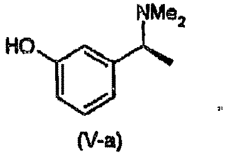

3. Prozess gemäß Anspruch 1 und 2, wobei Rivastigmin der Formel (I) gewonnen wird durch Verreagieren von (S)-3-(1-Dimethylaminoethyl)phenol der Formel V(a) mit Carbonylimidazol und Ethylmethylamin in einem organischen Lösungsmittel.

4. Prozess nach Anspruch 1 und 3, wobei die verwendete Menge Carbonyldiimidazol zwischen 1,5 Mol und 3,0 Mol pro Mol der Verbindung der Formel (V) oder Formel (Va) liegt.

5. Prozess nach Anspruch 4, wobei die verwendete Menge Carbonyldiimidazol bevorzugt zwischen 1,75 Mol und 2,25 Mol pro Mol der Verbindung der Formel (V) oder der Verbindung der Formel (V-a) liegt.

6. Prozess nach Anspruch 1 und 3, wobei die verwendete Menge Ethylmethylamin zwischen 1,0 Mol und 3,0 Mol pro Mol der Verbindung der Formel (V) oder der Verbindung der Formel (V-a) liegt.

7. Prozess nach Anspruch 6, wobei die verwendete Menge Ethylmethylamin bevorzugt zwischen 1,75 Mol und 2,25 Mol pro Mol der Verbindung der Formel (V) oder der Verbindung der Formel (V-a) liegt.

8. Prozess nach Anspruch 1b und nach Anspruch 3, wobei das verwendete Lösungsmittel aus der Gruppe ausgewählt wird, die aliphatischen Kohlenwasserstoff, Ester und chlorierte Lösungsmittel umfasst.

9. Prozess nach Anspruch 8, wobei das bevorzugte Lösungsmittel ein Chlorkohlenwasserstoff ist.

10. Prozess nach Anspruch 9, wobei das chlorierte Lösungsmittel aus der Gruppe ausgewählt wird, die Dichlormethan, Chloroform und Ethylendichlorid umfasst.

11. Prozess nach Anspruch 9, wobei der Chlorkohlenwasserstoff bevorzugt Dichlormethan ist.

12. Prozess nach Anspruch 1 oder Anspruch 3, wobei, sofern der Löseschritt nicht erforderlich ist, die Verbindung der Formel (I) von der Reaktionsmischung isoliert wird durch Löschen der Mischung mit Wasser, gefolgt von Extraktion mit einem organischen Lösungsmittel und Konzentration der organischen Schicht.

13. Prozess nach Anspruch 1, wobei für Schritt c) die Verbindung der Formel (I) von der Reaktionsmischung isoliert wird durch Löschen der Mischung mit Wasser, gefolgt von Konzentration der organischen Schicht, um die Verbindung der Formel (II) zu erhalten, die dann unter Verwendung einer optisch aktiven Säure wie D-Camphersulfonsäure gelöst wird.

14. Prozess zur Herstellung von Rivastigmin der Formel (I), umfassend die Reaktion von (S)-3-(1-Dimethylaminoethyl)phenol der Formel (V-a) mit einem Ethylmethylcarbamoylchlorid in einem inerten organischen Lösungsmittel bei erhöhter Temperatur und in der Anwesenheit einer Base, die aus der Gruppe ausgewählt wird, die Carbonate, Bicarbonate, Alkoxide von Alkali- oder Erdalkalimetallen umfasst, und das Isolieren der Verbindung der Formel (I).

15. Prozess nach Anspruch 14, wobei das organische Lösungsmittel aus der Gruppe ausgewählt wird, die aliphatische Kohlenwasserstoffe, Ester, Chlorkohlenwasserstoffe und Nitrile umfasst.

16. Prozess nach Anspruch 15, wobei das bevorzugte organische Lösungsmittel ein Nitril ist.

17. Prozess nach Anspruch 16, wobei das Nitril aus der Gruppe ausgewählt wird, die Acetonitril, Propionitril und Butyronitril umfasst.

18. Prozess nach Anspruch 17, wobei das bevorzugte Nitril Acetonitril ist.

19. Prozess nach Anspruch 18, wobei das Volumen des verwendeten Acetonitrils zwischen 10 und 15 Volumen pro Gramm der Verbindung (V-a) beträgt.

20. Prozess nach Anspruch 14, wobei die bevorzugte Base das Carbonat eines Alkalimetalls ist.

21. Prozess nach Anspruch 20, wobei die Base aus der Gruppe ausgewählt wird, die Lithiumcarbonat, Natriumcarbonat und Kaliumcarbonat umfasst.

22. Prozess nach Anspruch 21, wobei die bevorzugte Base Kaliumcarbonat ist.

23. Prozess nach Anspruch 22, wobei die verwendete Menge Kaliumcarbonat zwischen 1,0 und 4,0 Mol pro Mol der Verbindung (V-a) beträgt.

24. Prozess nach Anspruch 14, wobei die für die Reaktion verwendete Menge Ethylmethylcarbamoylchlorid zwischen 1,0 und 1,5 Mol pro Mol der Verbindung (V-a) beträgt.

25. Prozess nach Anspruch 14, wobei die Verbindung der Formel (I) aus der Reaktionsmischung isoliert wird durch Löschen der Mischung mit Wasser, gefolgt von Extraktion durch ein organisches Lösungsmittel und Konzentration der organischen Schicht.

## Revendications

1. Procédé de préparation de rivastigmine de formule (I) : 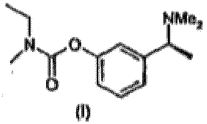 comprenant :
a) la réaction de 3-(1-diméthylaminoéthyl)phénol de formule (V) avec du carbonyldiimidazole 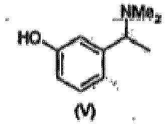
b) l'exécution d'une étape consistant à faire réagir un produit de l'étape a) avec de l'éthylméthylamine dans un solvant organique pour obtenir le composant (II) ; 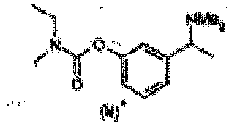
c) à titre facultatif la résolution du composé (II) pour donner le composé (I).

2. Procédé tel que revendiqué dans la revendication 1, où le 3-(1-diméthylaminoéthyl)phénol est le (S)-3-(1-diméthylaminoéthyl)phénol optiquement actif.

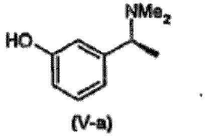

3. Procédé tel que revendiqué dans les revendications 1 et 2, la rivastigmine de formule (I) étant obtenue par réaction du (S)-3-(1-diméthylaminoéthyl)phénol de formule V(a) avec du carbonyle imidazole et de l'éthylméthylamine dans un solvant organique.

4. Procédé selon les revendications 1 et 3, la quantité de carbonyldiimidazole utilisée étant comprise entre 1,5 mole et 3,0 moles par mole de composé de formule (V) ou de formule (Va).

5. Procédé selon la revendication 4, la quantité de carbonyldiimidazole utilisée étant de préférence comprise entre 1,75 mole et 2,25 moles par mole de composé de formule (V) ou de composé de formule (V-a).

6. Procédé selon les revendications 1 et 3, la quantité d'éthylméthylamine utilisée étant comprise entre 1,0 mole et 3,0 moles par mole de composé de formule (V) ou de composé de formule (V-a).

7. Procédé selon la revendication 6, la quantité d'éthylméthylamine utilisée étant de préférence comprise entre 1,75 mole et 2,25 moles par mole de composé de formule (V) ou de composé de formule (V-a).

8. Procédé selon la revendication 1b et la revendication 3, le solvant utilisé étant choisi dans le groupe comprenant un hydrocarbure aliphatique, les esters et les solvants chlorés.

9. Procédé selon la revendication 8, le solvant préféré étant un hydrocarbure chloré.

10. Procédé selon la revendication 9, le solvant chloré étant choisi dans le groupe comprenant le dichlorométhane, le chloroforme et le dichlorure d'éthylène.

11. Procédé selon la revendication 9, l'hydrocarbure chloré étant de préférence du dichlorométhane.

12. Procédé selon la revendication 1 ou la revendication 3, dans lequel lorsque l'étape de résolution n'est pas requise, le composé de formule (I) est isolé du mélange réactionnel, par neutralisation à l'eau du mélange, puis extraction avec un solvant organique et concentration de la couche organique.

13. Procédé selon la revendication 1, dans lequel pour l'étape c), le composé de formule (I) est isolé du mélange réactionnel, par neutralisation du mélange à l'eau, puis concentration de la couche organique pour donner le composé de formule (II), qui est alors résolu en utilisant un acide optiquement actif comme l'acide (+)-camphorsulfonique.

14. Procédé de préparation de rivastigmine de formule (I) comprenant la réaction de (S)-3-(1-diméthylaminoéthyl)phénol de formule (V-a) avec un chlorure d'éthylméthyl carbomyle dans un solvant organique inerte à une température élevée et en présence d'une base choisie dans le groupe comprenant les carbonates, le bicarbonate, les alcoxydes de métaux alcalins ou alcalino-terreux et l'isolement du composé de formule (I).

15. Procédé selon la revendication 14, le solvant organique étant choisi dans le groupe comprenant les hydrocarbures aliphatiques, les esters, les hydrocarbures chlorés et les nitriles.

16. Procédé selon la revendication 15, le solvant organique préféré étant un nitrile.

17. Procédé selon la revendication 16, le nitrile étant choisi dans le groupe comprenant l'acétonitrile, le propionitrile et le butyronitrile.

18. Procédé selon la revendication 17, le nitrile préféré étant l'acétonitrile.

19. Procédé selon la revendication 18, le volume d'acétonitrile utilisé étant compris entre 10 et 15 volumes par gramme de composé (V-a).

20. Procédé selon la revendication 14, la base préférée étant le carbonate d'un métal alcalin.

21. Procédé selon la revendication 20, la base étant choisie dans le groupe comprenant le carbonate de lithium, le carbonate de sodium et le carbonate de potassium.

22. Procédé selon la revendication 21, la base préférée étant le carbonate de potassium.

23. Procédé selon la revendication 22, la quantité de carbonate de potassium utilisée étant comprise entre 1,0 et 4,0 moles par mole de composé (V-a).

24. Procédé selon la revendication 14, la quantité de chlorure d'éthylméthyle carbomyle utilisée pour la réaction étant comprise entre 1,0 et 1,5 mole par mole de composé (V-a).

25. Procédé selon la revendication 14, le composé de formule (I) étant isolé du mélange réactionnel par neutralisation du mélange à l'eau, puis extraction avec un solvant organique et concentration de la couche organique.
